(19) 

Europäisches
Patentamt
European
Patent Office
Office européen
des brevets

(11) **EP 2 416 144 A1**

(12) **EUROPEAN PATENT APPLICATION**
published in accordance with Art. 153(4) EPC

(43) Date of publication:
   **08.02.2012 Bulletin 2012/06**

(21) Application number: **10758732.1**

(22) Date of filing: **30.03.2010**

(51) Int Cl.:
   ***G01N 21/33*** (2006.01)      ***A61B 5/00*** (2006.01)
   ***A61K 8/00*** (2006.01)

(86) International application number:
   **PCT/JP2010/055729**

(87) International publication number:
   **WO 2010/113961 (07.10.2010 Gazette 2010/40)**

(84) Designated Contracting States:
   **AT BE BG CH CY CZ DE DK EE ES FI FR GB GR
   HR HU IE IS IT LI LT LU LV MC MK MT NL NO PL
   PT RO SE SI SK SM TR**

(30) Priority: **30.03.2009 JP 2009081400**

(71) Applicant: **Shiseido Company, Ltd.**
   **Chuo-ku**
   **Tokyo 104-8010 (JP)**

(72) Inventors:
   • **MIURA, Yoshimasa**
      **Yokohama-shi**
      **Kanagawa 224-8558 (JP)**

   • **MIZUNO, Hiroko**
      **Yokohama-shi**
      **Kanagawa 224-8558 (JP)**
   • **HARA, Yusuke**
      **Yokohama-shi**
      **Kanagawa 224-8558 (JP)**

(74) Representative: **Hart-Davis, Jason et al**
   **Cabinet Beau de Loménie**
   **158, rue de l'Université**
   **75340 Paris Cedex 07 (FR)**

(54) **APPLICATION METHOD OF EXTERNAL DERMATOLOGICAL MEDICATIONS, EVALUATING METHOD OF THE SAME, APPLICATION EVALUATING APPARATUS, AND APPLICATION EVALUATING PROGRAM**

(57)    A method of applying an external dermatological medication to a skin or a skin substitute substrate with an external dermatological medication for evaluating characteristics of the external dermatological medication acquired by irradiating the skin or the skin substitute substrate with light including a first applying step of spreading the external dermatological medication in one direction on the skin or the skin substitute substrate from a side surface of the skin or the skin substitute substrate; a second applying step of spreading the external dermatological medication in a direction perpendicular to the one direction on the skin or the skin substitute substrate, wherein the first applying step and the second applying step are repeated by a predetermined number of times.

FIG.6

```
        ┌─────────┐
        │  START  │
        └────┬────┘
             │  ┌──────────────┐
             ▼          S11
        ┌──────────────────┐
        │ ACQUIRING VALUE  │
        └────────┬─────────┘
                 │           S12
              ╱  WAS   ╲
            ╱ VALUE ACQUIRED ╲     NO
           ⟨ BY A PREDETERMINED ⟩────┐
            ╲  NUMBER OF   ╱         │
              ╲ TIMES? ╱             │
                 │ YES               │
                 │           S13     │
        ┌──────────────────┐        │
        │   CALCULATING     │        │
        │ EVALUATION VALUE  │        │
        └────────┬─────────┘        │
                 │           S14     │
        ┌──────────────────┐        │
        │  DETERMINATION    │        │
        └────────┬─────────┘        │
                 │                   │
        ┌────────▼────────┐         │
        │      END        │         │
        └─────────────────┘         │
```

EP 2 416 144 A1

**Description**

TECHNICAL FIELD

The present invention relates to a application method of external dermatological medications, an evaluating method of the application method, a application evaluating apparatus, and a application evaluating program. More specifically, it relates to a application method of external dermatological medications in which repeatability of applying the external dermatological medications is especially improved, an evaluating method of the application method, a application evaluating apparatus, and a application evaluating program.

BACKGROUND ART

As an example, external dermatological medications such as cosmetic products are provided to be applied to a skin basically with a finger or an applicator. An action of applying the external dermatological medications with the finger or the applicator can be freely adjustable depending on a time of use, a place of use, an occasion of use and/or a personal preference. Therefore, it is possible to provide a makeup cosmetic product to be thick makeup or thin makeup depending on a user's intention.

For the external dermatological medications used depending on the user's preference, if the application is easily adjustment, it is a great advantage for the user. However, if an ultraviolet radiation protection property is functionally obtained by a applying action, the functionality greatly depends on the applying action. Therefore, if a sufficient function is indispensable, the application adjustment needs to be handled in a careful manner.

Practically, a sun protection cosmetic product may be evenly spread on a skin with a finger. It is said that if the finger is used to spread the sun protection cosmetic product, depending on a way of spreading the sun protection cosmetic product with the finger, dispersibility of ingredients is improved to enhance an ultraviolet radiation protection effect. Further, according to an example in vivo SPF measurement method which is used to measure ultraviolet radiation protection effects of external dermatological medications, a finger or a finger cot is used to practically apply a human skin with external dermatological medications.

The above in vivo SPF value may be used as a scale indicating an ultraviolet radiation protection effect of external dermatological medications such as sun protection product for preventing sunburn caused by ultraviolet radiation. Specifically, the in vivo SPF value is an index of effects of protesting bare skin from sunburn caused by ultraviolet radiation and preventing the sunburn. The in vivo SPF value is defined by dividing an amount of ultraviolet radiation causing slight redness when an external dermatological medication is used by an amount of ultraviolet radiation causing slight redness when no external dermatological medication is used. For example, if a sun protection cosmetic product having an in vivo SPF value of 10 is used, sunburn is ten times less apt to occur in comparison with bare skin.

The in vivo SPF value may be measured by checking whether sunburn (erythema) occurs the next day after irradiating a bare skin which is not applied with an external dermatological medication and a bare skin applied with the external dermatological medication with a certain amount of ultraviolet radiation of artificial light (a solar simulator) which resembles solar light.

In use of the in vivo SPF value, ultraviolet radiation protection effects of external dermatological medications can objectively be evaluated. However, cooperation from a large number of test subjects having specific bare skin types is indispensable in order to measure the in vivo SPF value. Therefore, a great cost and many days are necessary for the measurement of the in vivo SPF value.

Patent Documents 1 to 3 disclose in vitro SPF evaluation methods, with which estimated in vitro SPF values are measured, without using test subjects. A skin substitute substrate used for the in vitro SPF evaluation method may be a polyethylene sheet, a nylon film (see Patent Document 4), a quartz plate, a PMMA plate (See Non-Patent Documents 1 and 2), and so on may be known. On one surface of the nylon film of Patent Document 1, a groove which models a furrow and has a V-like cross-sectional shape in the lateral direction of the groove is provided. On portions of the one surface of the nylon film other than the furrows, irregularity (concavity and convexity) is given by blast processing.

Meanwhile, Non-Patent Document 3 discloses that the application amount of an external dermatological medication used to measure an in vivo SPF value is 2.00 mg/cm$^2$. However, there is not a skin substitute substrate applied evenly with the external dermatological medication of the amount of 2.00 mg/cm$^2$. If a known skin substitute substrate is used, the application amount of an external dermatological medication in measuring an estimated in vitro SPF value is about 0.75 to 1.20 mg/cm$^2$.

Further, if an ultraviolet absorber contained in an external dermatological medication is degraded by ultraviolet radiation, the ultraviolet absorber is progressively degraded at a time of measuring an in vivo SPF value. Therefore, the estimated in vitro SPF values of the external dermatological medications containing these ultraviolet absorbers may not sufficiently reproduce measurement conditions of the in vivo SPF value because the application amounts of the external dermatological medications used in measuring the in vitro SPF values are different from those used in measuring the in vivo SPF values. It is important to set the amount (2.00 mg/cm$^2$) of applying the external dermatological medication in measuring the estimated in vitro SPF value to be the same as the amount of applying the external dermatological medication in measuring the in vivo SPF value. By using the same amounts, measurement conditions of the in vivo SPF value are reproduced not only to compensate for the progressive degradation of the ultraviolet absorber with the ultraviolet radiation but also to reproduce the conditions of applying the external dermatological medication in a microscopic level.

[Patent Documents]

[Patent Document 1] Japanese Patent No. 3337832
[Patent Document 2] Japanese Laid-open Patent Publication No. 2008-96151
[Patent Document 3] Japanese Laid-open Patent Publication No. 2008-111834
[Patent Document 4] Japanese Laid-open Patent Publication No. 2002-48789

[Non-Patent Documents]

[Non-Patent Document 1] Ferrero L. et al., Importance of Substrate Roughness for In vitro Sun Protection Assessment, IFSCC Magazine,Vol.9,No.2,2-13(2006)
[Non-Patent Document 2] COLIPA GUIDELINES, METHOD FOR THE IN VITRO DETERMINATION OF UVA PROTECTION PROVIDED BY SUNSCREEN PRODUCTS, Edition of 2007
[Non-Patent Document 3] International Sun Protection Factor Test Method, (C.O.L.I.P.A.-J.C.I.A.-C.T.F.A.S.A.-C.T.F.A. ),May 2006

DISCLOSURE OF THE INVENTION

PROBLEMS TO BE SOLVED BY THE INVENTION

However, in a conventional technique, a applied state of external dermatological medications in a microscopic level has not been standardized, and measured values vary (becomes uneven) depending on the applied state. Therefore, an accuracy of the evaluation has been affected.

For example, in a research institute actually carrying out in vivo SPF measurements, trainees by themselves traditionally make efforts of reducing unevenness of application upon instructions from skilled personnel. There are not a clear method and a training procedure for improving application repeatability. Further, there is not a tool and the like for determining what kind of training is required for an applicator to realize a predetermined applied state.

The embodiments of the present invention are provided in consideration of the above problems. The objects of the embodiments are to provide a application method of external dermatological medications, an evaluating method of the application method, a application evaluating apparatus, and a application evaluating program.

MEANS FOR SOLVING PROBLEMS

In order to solve the problems, the present invention adopts measures for solving the problems having the following features.

According to a first aspect of the present invention, there is provided a method of applying a skin or a skin substitute substrate with an external dermatological medication for evaluating characteristics of the external dermatological medication acquired by irradiating the skin or the skin substitute substrate with light, the method including a first applying step of spreading the external dermatological medication in one direction on the skin or the skin substitute substrates from a side surface of the skin or the skin substitute substrate; and a second applying step of spreading the external dermatological medication in a direction perpendicular to the one direction on the skin or the skin substitute substrate, wherein the first applying step and the second applying step are repeated by a predetermined number of times.

The wavelength region in the present invention is not specifically limited. For example, when an outer appearance of an appl ying state is checked under room light or sunlight, the present invention is effective. With this, an application repeatability in applying a dermatological medication to a skin or a skin substitute substrate can be improved.

Further, with the even application of the present invention, various properties of the external dermatological medications on the skin substitute substrate can be highly accurately evaluated. For example, the present invention can also be used to evaluate repeatability of physical properties without emitting light such as water repellency and oil repellency on the skin.

According to a second aspect of the present invention, there is provided a time for the first applying step and the second applying step multiplied by the predetermined number of times results in about 3 0 seconds to about 90 seconds after starting the first applying step or the second applying step, and a number of strokes for applying to the skin or the skin substitute substrate per about one second is 0. 5 to 5 and one cycle including the first applying step and the second applying step is about 3 seconds to about 20 seconds.

According to a third aspect of the present invention, there is provided an evaluating method of the skin or the skin substitute substrate applied by the method of applying the external dermatological medication to the skin or the skin substitute substrate including acquiring a value by changing a transmitted light intensity or a transmittance, or a reflected light intensity or a reflectance, which are acquired by irradiating the skin or the skin substitute substrate with the light; calculating an evaluation value for carrying out at least one of a applied surface unevenness evaluation, an unevenness evaluation between applied substrates, an absolute value evaluation using a simple in vitro SPF, and a final value evaluation using an in vitro SPF evaluating apparatus by acquiring the values acquired by the acquiring the value for plural times; and determining whether applying the external dermatological medication to the skin or the skin substitute substrate is accurately carried out based on the evaluation value calculated by the calculating the evaluation value.

With this, application repeatability in applying the external dermatological medications can be improved.

According to a fourth aspect of the present invention, the calculating the evaluation value calculates a CV evaluation value of distribution of the simple in vitro SPFs as the unevenness evaluation between applied substrates.

According to a fifth aspect of the present invention, a 17% screening of distribution of the simple in vitro SPF values is carried out as the unevenness evaluation between applied substrates.

According to a sixth aspect of the present invention, there is provided a application evaluating apparatus for evaluating characteristics of an external dermatological medication acquired by irradiating the external dermatological medication with light after applying the external dermatological medication to the skin or a skin substitute substrate including a value acquiring unit configured to acquire a value by changing a transmitted light intensity or a transmittance, or a reflected light intensity or a reflectance, which are acquired by irradiating the skin or the skin substitute substrate with the light; an evaluation value calculating unit configured to calculate an evaluation value for carrying out at least one of a applied surface unevenness evaluation, an unevenness evaluation between applied substrates, an absolute value evaluation using a simple in vitro SPF, and a final value evaluation using an in vitro SPF evaluating apparatus by acquiring the values acquired by the value acquiring unit for plural times; and a determining unit configured to determine whether applying the external dermatological medication to the skin or the skin substitute substrate is accurately carried out based on the evaluation value calculated by the calculating the evaluation value.

With this, application repeatability in applying the external dermatological medications can be improved.

According to a seventh aspect of the present invention, the evaluation value calculating unit calculates a CV evaluation value of distribution of the simple in vitro SPFs as the unevenness evaluation between applied substrates.

According to an eighth aspect of the present invention, the evaluation value calculating unit carries out a 17% screening of distribution of the simple in vitro SPF values is carried out as the unevenness evaluation between applied substrates.

According to a ninth aspect of the present invention, there is provided a application evaluating program for evaluating characteristics of an external dermatological medication acquired by irradiating the external dermatological medication with light after applying the external dermatological medication to the skin or a skin substitute substrate, the evaluating program causing a computer to function as: a value acquiring unit configured to acquire a value by changing a transmitted light intensity or a transmittance, or a reflected light intensity or a reflectance, which are acquired by irradiating the skin or the skin substitute substrate with the light; an evaluation value calculating unit configured to calculate an evaluation value for carrying out at least one of a applied surface unevenness evaluation, anunevenness evaluationbetween applied substrates, an absolute value evaluation using a simple in vitro SPF, and a final value evaluation using an in vitro SPF evaluating apparatus by acquiring the values acquired by the value acquiring unit for plural times; and a determining unit configured to determine whether applying the external dermatological medication to the skin or the skin substitute substrate is accurately carried out based on the evaluation value calculated by the calculating the evaluation value.

With this, application repeatability in applying the external dermatological medications can be improved. Further, by installing the program in a computer, a result of analyzing a questionnaire in the present invention can be easily realized by the computer such as a general-purpose computer.

According to a tenth aspect of the present invention, the evaluation value calculating unit calculates a CV evaluation value of distribution of the simple in vitro SPFs as the unevenness evaluation between applied substrates.

According to an eleventh aspect of the present invention, the evaluation value calculating unit carries out a 17% screening of distribution of the simple in vitro SPF values is carried out as the unevenness evaluation between applied substrates.

EFFECT OF THE INVENTION

According to the embodiments of the present invention, repeatability in applying external dermatological medications can be improved.

BRIEF DESCRIPTION OF DRAWINGS

FIG. 1 illustrates an example flowchart for illustrating a application method of embodiments.
FIG. 2 illustrates an example way of spreading an external dermatological medication of the embodiments.
FIG. 3 illustrates an example application evaluating apparatus for evaluating permeation characteristics of ultraviolet radiation of an external dermatological medication of the embodiments.
FIG. 4 illustrates an example functional structure of a application evaluating apparatus of the embodiments of the present invention.
FIG. 5 illustrates an example hardware structure enabling an evaluation process of the embodiment of the present invention.
FIG. 6 illustrates an example flowchart for illustrating an example application evaluating procedure of the embodiments.
FIG. 7A illustrates contents of evaluations of the embodiments.
FIG. 7B illustrates contents of evaluations of the embodiments.
FIG. 7C illustrates contents of evaluations of the embodiments.
FIG. 8 illustrates an example determination result using a training determination tool of the embodiment.

BEST MODE FOR CARRYING OUT THE INVENTION

(SUMMARY OF THE INVENTION)

According to the embodiments of the present invention, when predetermined portions of a skin, a skin substitute substrate and so on are applied with an external dermatological medication such as a cosmetic product, knowhow and experience of skilled personnel are formalized as knowledge. Then, the embodiments systematically provide a clear method and a training procedure so that a person having no experience of applying an external dermatological medication to a skin substitute substrate and so on can attain high application repeatability within a very short time. With this, application repeatability is enhanced to thereby resultantly enhance repeatability of data of in vitro SPF measurements and so on.
Hereinafter, modes of preferably carrying out a application method of external dermatological medications, an evaluating method of the application method, a application evaluating apparatus, and a application evaluating program of the embodiments of the present invention are described with reference to figures. In the following description of the embodiments, a application method of an external dermatological medication using a skin substitute substrate (a plate) is explained. However, the present invention is also applicable to skin. Further, in the embodiments, a finger with a finger cot may be used in applying an external dermatological medication to the skin substitute substrate in compliance with a standard of measuring in vivo. However, the embodiments of the present invention are not limited to the use of the finger cot.
Further, in the following description, a sunscreen (anti-sunburn) cosmetic product is exemplified. However, the present invention is not limited thereto. For example, makeup cosmetics, skin-care cosmetics, pre-makeup cosmetics, body-care cosmetics and so on may be used as external dermatological medications. Forms of the external dermatological medication are not specifically limited. For example, emulsion, lotion, solid form, oil, spray and so on may be used.

(Application method)

A application method of a sunscreen agent (a cosmetic product) is described with reference to figures. The following skin substitute substrate is used in the embodiment.
[Material]: The material of the skin substitute substrate may be polymethylmethacrylate (PMMA) and has a transmittance of light of 290 to 400 nm of 50% or more to 100% or less.
[Thickness]: The thickness of the skin substitute substrate is 100 microns to 5000 microns (0.1 mm to 5.0 mm).
[Surface shape]: The surface shape of the skin substitute substrate includes furrows. The furrows are shaped like a V in their cross-sectional shapes, have depths of 30 to 150 $\mu$m (micron), widths of 50 to 500 $\mu$m (micron), frequencies on the longitudinal and lateral directions of 0.1 to 2.0 lines/mm, and a frequency on the diagonal 45º direction of 0.1 to 2.0 lines/mm.

Further, portions corresponding to ridges of the skin substitute substrate have arithmetic average surface roughness of 0.1 to 30 $\mu$m (micron). The shapes modeling the furrows and the ridges are provided only on one surface of the skin substitute substrate.

[Surface scattering characteristics]: When the skin substitute substrate is applied with glycerin by a predetermined amount (1 mg/cm$^2$), a transmittance difference between before and after applying glycerin to the skin substitute substrate in all wavelengths between 290 to 400 nm is 20% or less.

Conditions of the skin substitute substrate used in the embodiments of the present invention are not limited to the above.

(Application method of equalizing applying a sunscreen agent as an external dermatological medication)

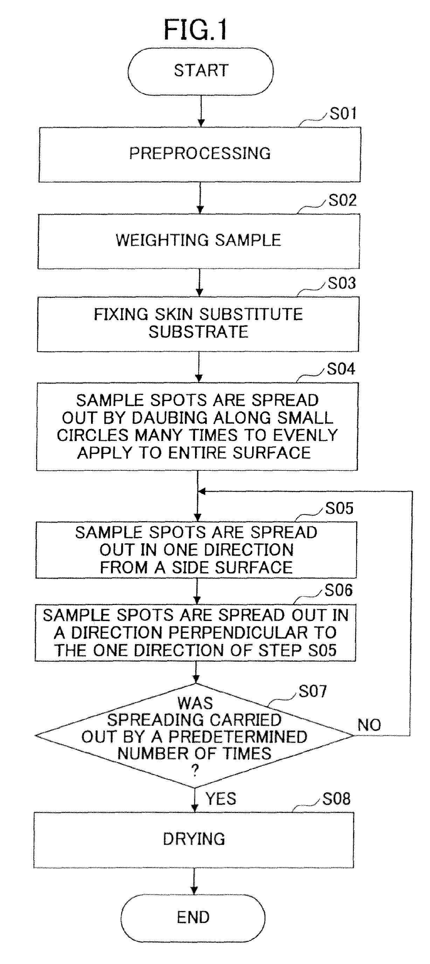

Next, a concrete method of applying a sunscreen agent to the skin substitute substrate in an in vitro SPF evaluation is described with reference to a flowchart. FIG. 1 illustrates an example flowchart for illustrating a application method of the embodiments.

Referring to FIG. 1, a preprocessing is carried out to weigh a sample in step S01. For example, in the preprocessing, a skin substitute substrate having dimensions of about 5×5 cm is set in an electronic balance, a zero point adjustment is done for the electronic balance, and a sample (the sunscreen agent) to be measured is mixed well. It is preferable to wear a finger cot on the index finger of a dominant hand before weighing a sample. In order to quickly move from the weighing of the sample to the application, the finger cot is properly worn without causing wrinkles. This is because the sample may be caught between the wrinkles and therefore an accurate amount of the sample may not be applied.

Next, the sample is weighed in step S02. Specifically, a syringe or the like is used to weigh the sample (for example, 50 seconds). For example, the weighing of the sample is preferably in a range of about 10 to 80 spots per skin substitute substrate having a size of 5 cm × 5cm, more preferably about 30 to 60 spots per the size of 5 cm × 5 cm. If the weighing of the sample is less than 10 spots per the size of 5 cm × 5cm, it becomes difficult to evenly apply to the surface of the skin substitute substrate. If the weighing of the sample is more than 80 spots per the size of 5 cm × 5cm, a time necessary for weighing the sample may elapse thereby causing application unevenness of the sample. Spot positions where the sample is placed in applying the sample are preferably even to the entire applied surface of the skin substitute substrate. Because there may be a sample with high volatility, it is important to apply the sample to the skin substitute substrate in a predetermined time set for weighing the sample as accurate as possible. Immediately after weighing the sample, the sample is applied.

Specifically in the procedure of applying the sample, the skin substitute substrate is fixed in step S03, and the sample is spread out by daubing along small circles many times to evenly apply to the entire surface in step S04. In step S03, four coroners of the skin substitute substrate is fixed to a table with a double-faced adhesive tape to prevent the skin substitute substrate from moving during the application. It is preferable to use a blackish color for the table used for applying the sample and a jig for fixing the skin substitute to the table. This is because application evenness can be being visually checked while the sample is applied.

Furthermore, an applicator who applies the sample to the skin substitute substrate preferably retains a ramrod-straight posture and an armpit closing posture and uses a tip portion of a finger next to the finger pad to make the tip portion contact the skin substitute substrate. The applicator preferably learns an actual motion of a finger by watching a motion picture such as video, a demonstration by a skilled personnel, and/or the like before the applicator trains and applies the sample such as a sunscreen agent to the skin substitute substrate.

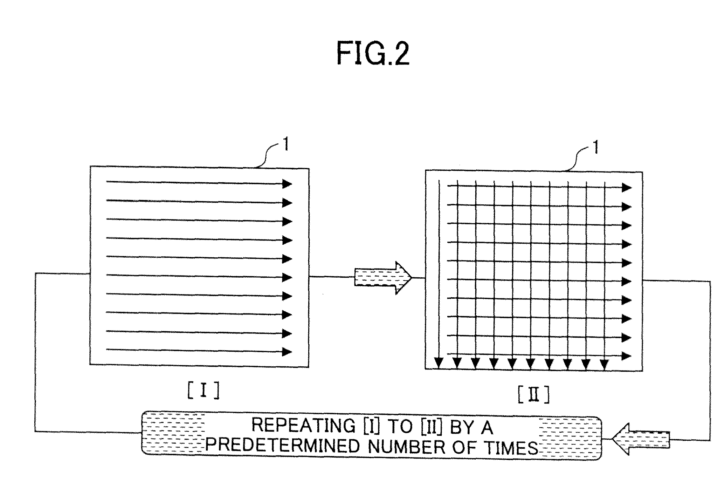

With the embodiment, the following method is basically used to spread the sample in step S05. FIG. 2 is an example way of spreading an external dermatological medication of the embodiment.

As the first step of an applying procedure, the sample is spread on a skin substitute substrate 1 from one side surface in one direction (from left to right in FIG. 2) as illustrated in [I] of FIG. 2 in step S05. As the second step of the applying procedure, after the sample is spread over the entire surface of the skin substitute substrate 1, the sample is spread over the surface of the skin substitute substrate 1 in a direction perpendicular to (different from by 90º) the one direction (from up to down in FIG. 2) as illustrated in [II] of FIG. 2 in step S06. In step S06, the sample is spread over the entire surface of the skin substitute substrate 1 in a similar manner to step S05.

Subsequently, it is determined whether step S05 and step S06 are carried out by a predetermined number of times in step S07. If it is not carried out by the predetermined number of times in NO of step S07, the process returns to step S05 and subsequent processes are carried out. If step S05 and step S06 are carried out by the predetermined number of times in YES of step S07, a drying process is carried out in step S08 and the process ends.

In step S07, the processes [I] and [II] illustrated in FIG. 2 are combined to be one cycle, and the combined processes are carried out by plural times (a repeating procedure). For example, the number of the repeating procedures is within a range of about 3 to 10 times. This is because the predetermined time limit is applied to the application time.

If the number of the repeating procedures is up to two, an application speed is too slow to sufficiently dry the sample thereby preventing from evenly applying the sample to the skin substitute substrate. If the number of the repeating procedures is more than ten, an application speed is very fast . Then, the sample is quickly dried and the application is repeated more than necessary, and therefore the skin substitute substrate cannot be evenly applied. The number of the repeating procedures is preferably about 4 to 8 times.

(Detailed application method)

A detail of the above-described application method is described. The time for entirely applying the sample to the skin substitute substrate in step S04 is preferably about 3 to 20 seconds, specifically about 10 seconds. If the time is 3 seconds or less, the sample spots located at the time of weighing the sample do not sufficiently spread over the entire surface of the skin substitute substrate. Meanwhile, if the time is more than 20 seconds, a time for spreading over the entire surface of the skin substitute substrate in the subsequent process of step S05 is shortened. Therefore, the application resultantly becomes uneven.

Here, if 20 strokes (corresponding to the number of arrows in FIG. 2) are provided to spread the sample in a ratio of 2 strokes per one second, a time for the one cycle is about 10 seconds. Said differently, the application of steps S05 and S06 ends within about one minute. With the embodiment, the number of strokes per the cycle and the time of the one cycle are adjusted as described above.

(Notice for application)

The applicator maintains force for spreading the sample with his or her hand to be constant in conformity with the above described application method. Specifically, the finger (the index finger) of the applicator applying the sample may be just in contact with the skin substitute substrate. Further, the SPF value may largely change depending on the force applied to the skin substitute substrate. Therefore, the applicator is always required to maintain constant conditions. The finger can daub the sample back and forth onto the skin substitute substrate to spread the sample. However, in this case, the sample is apt to gather around side surfaces to cause unevenness of the sample. Therefore, it is preferable not to daub the sample back and forth onto the skin substitute substrate with the finger and to spread the sample in one direction only.

Further, the application ends after a time range of about 40 to 80 seconds passes from starting to spread the sample. However, if the application unevenness is checked by visually evaluating an outer appearance of the skin substitute substrate, the application may end within about several seconds after the predetermined time. If the time range is less than 40 seconds, the entire surface of the skin substitute substrate may be unevenly applied with the sample. If the time range is more than 80 seconds, the skin substitute substrate may be excessively applied with the sample. Then, in a similar manner to the time range of less than 40 seconds, the entire surface of the skin substitute substrate may be unevenly applied with the sample. The time range from the start and end of the application is preferably about 1 minute.

Although the above embodiment describes spreading in the one direction and spreading in the perpendicular direction several times, the present invention is not limited thereto. For example, the sample may be spread by daubing the sample along circles gradually from the center of the skin substitute substrate to the periphery of the skin substitute substrate. Further, if the skin substitute substrate is not fixed to a flat obj ect such as a table by an adhesive tape, after step S05 of FIG. 1, the skin substitute substrate may be rotated by an integral multiple of 90º in step S06 and the sample may be daubed every integral multiple of 90º in corresponding one directions.

(Drying)

Step S08 of the above described applying procedure is described. In step S08, the samples on the skin substitute substrates are dried under identical conditions every time by accommodating in a dark place having a room temperature for 15 minutes from the end of the application. After the end of the drying process, SPFs are evaluated.

With the even application of the embodiment, it is possible to highly accurately evaluate various properties of external dermatological medications on skin substitute substrates. For example, the embodiment is applicable to evaluation of repeatability of physical properties without emitting light such as water repellency and oil repellency.

Next, an application evaluating technique of evaluating whether the skin substitute substrate is appropriately applied with the external dermatological medications by the above applying procedure is described with reference to figures.

(Exemplary application evaluating apparatus)

For example, a application evaluating apparatus may be an evaluating apparatus for evaluating permeation characteristics and/or reflection characteristics of external dermatological medications. The application evaluating apparatuses disclosed in Patent Document 2 (Japanese Laid-open Patent Publication No. 2008-96151) and Patent Document 3 (Japanese Laid-open Patent Publication No. 2008-111834) may be used. However, the application evaluating apparatus is not limited thereto. By using these apparatuses, a sample can be measured while continuously irradiating with light in a similar manner to an in vivo SPF measurement. Even i f a photodegradation phenomenon or the like occurs in the sample, it is possible to acquire an in vitro SPF value with a principle similar to an in vivo SPF measurement. On the other hand, although it is not possible to continuously irradiate light in a similar manner to the in vivo SPF measurement, an ordinary spectrophotometer, an SPF analyzer and so on can be used as an apparatus for evaluating the above applied state. The ordinary spectrophotometer can measure in a wavelength region of 290 nm to 400 nm which is necessary in making predictions of SPF. The SPF analyzer (UV-1000S and UV-2000S manufactured by LABSPHERE, INC.) is developed for making predictions of in vitro SPF values using the same principle as the spectrophotometer and can evaluate the in vitro SPF value in a simple way. Here, measurement and analysis principles in the simple way are different from those of the above described in vitro SPF.

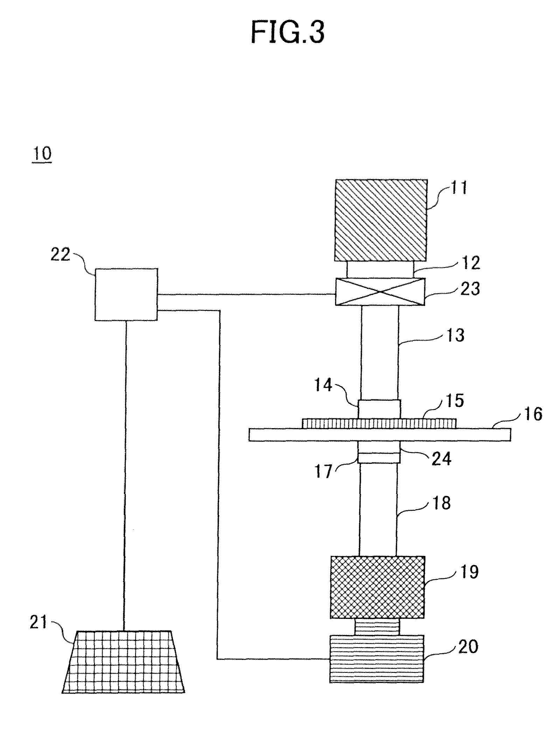

FIG. 3 illustrates an example application evaluating apparatus for evaluating permeation characteristics of an ultraviolet radiation of an external dermatological medication of the embodiments. The application evaluating apparatus 10 includes a light source 11, a filter 12, an optical fiber 13, an irradiationport 14, a skin substitute substrate applied with an external dermatological medications 15, a detection port 17, an optical fiber 18, a spectrometer 19, a photodetector 20, an electric signal and application evaluating processing unit (a computer 21), a lock-in amplifier 22, an optical chopper 23, and an integrating sphere 24.

The light source 11 is not specifically limited. For example, the light source 11 may be a xenon lamp or the like being a light source of a white light for emitting an ultraviolet radiation, a visible radiation, an infrared radiation, and so on. The white light emitted by the xenon lamp can be used as a simulated sunlight.

The filter 12 is positioned in the vicinity of a light travelling direction of a light emitted from the light source 11, and corrects the emitted light to be a ultraviolet radiation such as a ultraviolet radiation having a wavelength of 290 to 400 nm. The corrected ultraviolet radiation is incident on the optical chopper 23. The filter 12 is not specifically limited. For example, WG320 and UG 11 manufactured by SCHOTT AG or the like can be used.

The optical chopper 23 may be a shutter intermittently allowing the ultraviolet radiation through it for pulse-irradiating the ultraviolet radiation. The pulse-irradiated ultraviolet radiation is emitted into the optical fiber 13. The optical chopper 23 is electrically connected to the lock-in amplifier 22. A synchronization signal of the pulse-irradiatedultraviolet radiation is obtained from the lock-in amplifier 22 or a driving circuit for synchronously analyzing the signal from the photodetector 20.

The optical fiber 13 is positioned in the vicinity of the light travelling direction of the ultraviolet radiation emitted from the optical chopper 23 and guides the ultraviolet radiation to the irradiation port 14. A skin substitute substrate 16 applied with an external dermatological medication 15 is irradiated by the ultraviolet radiation guided to the irradiation port 14.

The irradiation port 14 and the detection port 17 are fixed while interposing a predetermined interval between these. The skin substitute substrate 16 applied with the external dermatological medication 15 is fixed while interposing a predetermined gap from the irradiation port 14. At this time, the irradiation port 14, the external dermatological medication 15, the skin substitute substrate 16 and the integrating sphere 24 are arranged in this order along the light traveling direction of the ultraviolet radiation.

The skin substitute substrate 16 corresponds to the above skin substitute substrate 1. When necessary, the skin substitute substrate 16 may be arranged on a substrate made of quartz or the like which is excellent in permeation characteristics of ultraviolet radiation.

The detection port receives the ultraviolet radiation uniformed by the integrating sphere 24 and guides the received radiation into the optical fiber 18. The optical fiber 18 is located in the vicinity of the light travelling direction of the ultraviolet radiation emitted from the detection port 17, and guides the ultraviolet radiation received by the detection port 17 into the spectrometer 19.

The spectrometer 19 separates the light having a range of 290 to 400 nm included in the ultraviolet radiation emitted from the optical fiber 18 with an interval of 1 nm. The ultraviolet radiation separated by the spectrometer 19 is incident on the photodetector 20.

The sensitivity characteristic of the spectrometer 19 is adjusted for the ultraviolet radiation. By using a diffraction grating especially having excellent sensitivity characteristics in the range of 290 to 400 nm, the light can be separated with high sensitivity. The diffraction grating may be a concave diffraction grating (type 10-015) manufactured by Shimadzu Corporation. However, the diffraction grating is not specifically limited to this.

The photodetector 20 detects the ultraviolet radiation separated by the spectrometer 19 with an optical sensor.

The photodetector 20 converts the intensity of the light beams having various wavelengths into signals of electric current or voltage. The current or voltage signals are transmitted to the computer 21 connected by electrical wiring.

The photodetector 20 is adjusted to have the sensitivity characteristics in the ultraviolet radiation. By using a photomultiplier especially having the sensitivity characteristics in a range of 290 to 400 nm, the sensitivity of detecting the ultraviolet radiation can be improved. The photomultiplier may have a photoelectric surface made of In, Ga, N, Al, O, Cs, and so on, specifically the photomultiplier may have an InGaN photoelectric surface and so on. However, the photomultiplier is not limited thereto. The photodetector 20 may be a semiconductor photodetector made of In, Ga, N, Al, O, and so on.

The computer 21 is electrically connected to the lock-in amplifier 22. The computer 21 receives data acquired by detecting and processing with the lock-up amplifier 22 the signal received from the photodetector 20, and processes the data so as to be easily understood by a user of the application evaluating apparatus. The result of the processed data is to be printed on a recoding paper or stored in a recording medium by the computer 21. Further, the computer 21 evaluates application of the embodiments of the present invention.

The computer 21 may be a general-purpose personal computer or the like. Upon an instruction from a user via an input unit or the like, various functions of the application evaluating apparatus 10 can be carried out.

The lock-in amplifier 22 is electrically connected to the photodetector 20, the computer 21, and the optical chopper 23. The lock-in amplifier 22 controls the pulse-radiation received from optical chopper 23 and the signal received from the photodetector 20 so that the pulse radiation is in synchronism with the signal. Specifically, a phase detector circuit included in the lock-in amplifier 22 is used to synchronize the pulse-radiation and the received signal.

The integrating sphere 24 receives the ultraviolet radiation that passes through the external dermatological medication 15 and the skin substitute substrate 16, converges the ultraviolet radiation, and spatially integrates the ultraviolet radiation to make it uniform. The integrating sphere 24 may be omitted. When necessary, the of the ingredients inside the application evaluating apparatus 10 can be changed.

For example, an evaluating method of permeation characteristics of ultraviolet radiation of the external dermatological medication may be an in vitro SPF (SUN PROTECTION FACTOR) evaluation methods disclosed in Patent Document 1 (Japanese Patent No. 3337832) and Patent Document 3 (Japanese Laid-open Patent Publication No. 2008-111834). However, the evaluating method is not specifically limited thereto. With this, the estimated in vitro SPF value can be measured.

Besides the above evaluating method, an evaluating method of permeation characteristics and/or reflection characteristics of the ultraviolet radiation of the external dermatological medication may be an in vitro UVA evaluation method , an in vitro PPD method, an in vitro PFA method, a Critical Wavelength method, a UVA/UVB ratio method, an Australian/New Zeal and method, a German DIN UVA balance method, an SPF/UVAPF (PPD) ratio method, and so on. A combination of these methods may also be used as in Non-Patent Document 1 (Ferrero L.et al., Importance of Substrate Roughness for In vitro Sun Protection Assessment, IFSCC Magazine,Vol.9,No.2,2-13(2006)).

The wavelength region in the embodiments of the present invention may not be limited to plural wavelengths exemplified in the embodiments. For example, a room light and sunlight may be used which are effective for checking the applied state of the skin substitute substrate with the external dermatological medication in its appearance. As described, application repeatability in applying external dermatological medications to skins or skin substitute substrates can be improved.

(An exemplary functional structure of the application evaluating apparatus 10)

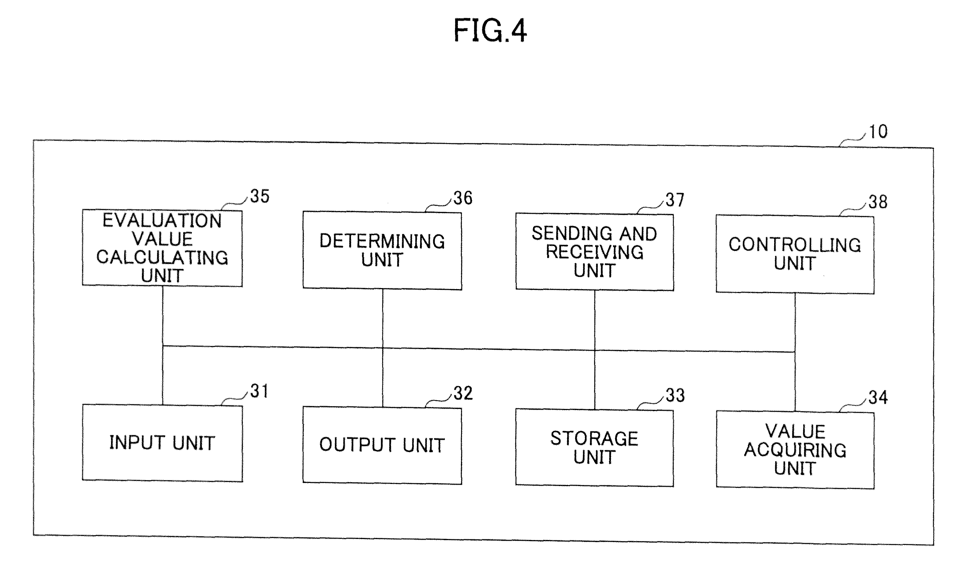

Next, the exemplary functional structure of the application evaluating apparatus 10 is described with reference to figures. FIG. 4 illustrates an example functional structure of the application evaluating apparatus of the embodiments of the present invention. As illustrated in FIG. 4, the application evaluating apparatus 10 includes an input unit 31, an output unit 32, a storage unit 33, a value acquiring unit 34, an evaluation value calculating unit 35, a determining unit 36, a sending and receiving unit 37 and a control unit 38.

For example, the input unit 31 is provided in the computer 21 and receives inputs requesting to start and stop a value acquiring process, an evaluation value calculating process, a determining process and so on. The input unit 31 includes a keyboard, a pointing device such as a mouse, and so on.

The output unit 32 is provided in, for example, the computer 21, and displays or outputs contents input through the input unit 31 or the contents of what is executed based on the input contents. The output unit 32 includes a display, a speaker and so on.

For example, the storage unit 33 is provided in the computer 21. The storage unit 33 stores data as a result of acquiring values with the value acquiring unit 34, a calculation result acquired by the evaluation value calculating unit, a determination result obtained by the determination unit 36, and so on.

The value acquiring unit 34 acquires a SPF evaluation value 34 evaluated by the application evaluating apparatus. Hereinafter, the values may be acquired to evaluate the permeation characteristics and/or reflection characteristics of the ultraviolet radiation of the external dermatological medications. Said differently, the value acquiring unit 34 changes the transmitted light intensity, the transmittance obtained by irradiating the skin substitute substrate with light, the reflected light intensity, or the reflectance to the values and acquires the values.

The evaluation value calculating unit 35 acquires the values acquired by the value acquiring unit 34 by plural times and calculates evaluation values used for carrying out a predetermined evaluation based on the plural acquired values. Specifically, the evaluation value calculating unit 35 calculates an evaluation value used to carry out at least one of a applied surface unevenness evaluation, an unevenness evaluation between applied substrates, an absolute value evaluation using simple in vitro SPF, and a final value evaluation using an in vitro SPF evaluating apparatus. In the applied surface unevenness evaluation, the evaluation value calculating unit 35 may calculate a CV evaluation value of unevenness in the simple in vitro SPF values and/or carry out a 17% screening of unevenness using the simple in vitro SPF values in order to realize the applied surface unevenness evaluation, the unevenness evaluation between applied substrates, the absolute value evaluation using the simple in vitro SPF, and the final value evaluation using the in vitro SPF evaluating apparatus.

Hereinafter, the 17% screening (+17% and -17% screening) is in conformity with a method disclosed in Non-Patent Document 3, Appendix IV. Said differently, if confidence limits (95% Confidence Interval) (95%CIn'[%]) for a mean SPF calculated based on simple in vitro SPF values, for each skin substitute substrate, falls within the range of $\pm$ 17% of the mean SPF, it is determined to be "pass" .

However, in the embodiments of the present invention, it is possible to adopt methods other than the applied surface unevenness evaluation, the unevenness evaluation between applied substrates, the absolute value evaluation using the simple in vitro SPF, and the final value evaluation using the in vitro SPF evaluating apparatus.

The determining unit 36 determines whether the skin substitute substrates are properly applied with the external dermatological medications based on the evaluation value calculation result obtained by the evaluation value calculating unit 35. By determining with the determining unit 36, even if an applicator has no experience of applying an external dermatological medication, it is possible to achieve sufficient application repeatability within a very short time.

For example, the sending and receiving unit 37 may be connected to a communication network such as the Internet with a small computer system interface (SCSI) cable, a universal serial bus (USB) cable, and a local area network (LAN) cable. Then, an execution program is connected to the communication network to enable acquiring the execution program from another terminal which can send and receive data via the communication network, providing an execution result obtained by executing the execution program to the other terminal, and providing the execution program causing a computer to realize the embodiment of the present invention to the other terminal. Further, the sending and receiving unit 37 acquires the SPF evaluated values of the external dermatological medications such as cosmetic products applied to the skin substitute substrates using the value acquiring unit 34. The acquired values are stored in the storage unit 33.

The control unit 38 controls the application evaluating apparatus 10 in its entirely. Specifically, the control unit 38 controls the value acquiring processes, the evaluation value calculating processes, the determining processes, and so on based on the instructions of the users via the input unit 31. With this, application repeatability is enhanced to thereby resultantly enhance repeatability of data of in vitro SPF measurements and so on.

(An exemplary hardware configuration of the application evaluating apparatus 10)

The above described application evaluating apparatus 10 is realized by generating execution programs (application evaluating program) enabling carrying out the various functions with a computer, and installing the generated execution program into a general purpose computer, a server or the like.

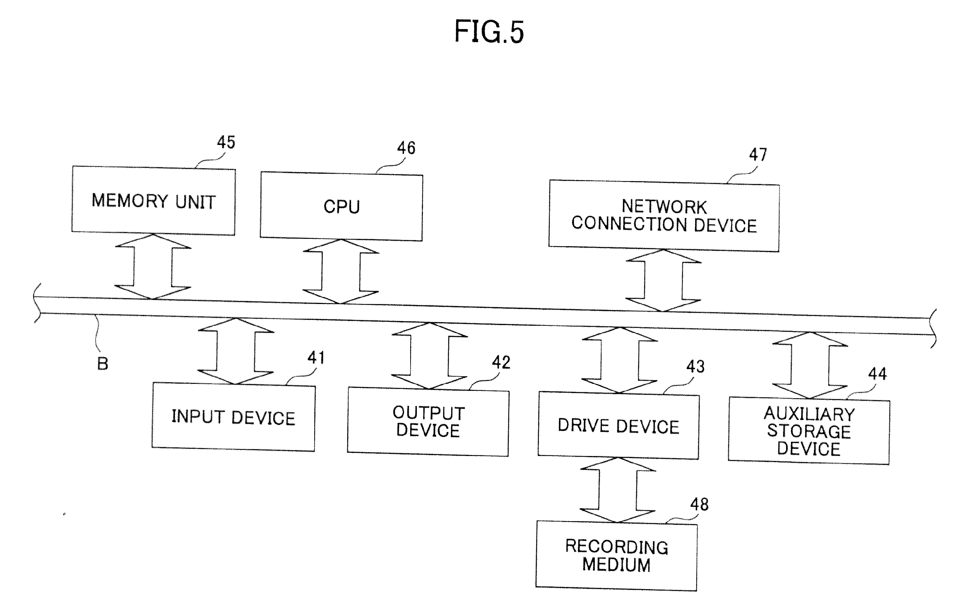

The hardware configuration of the computer which may carry out the application evaluating process of the embodiment of the present invention is described with reference to figures. FIG. 5 illustrates the hardware configuration enabling the application evaluating process of the embodiment of the present invention, as an example.

The computer illustrated in FIG. 5 includes an input device 41, an output device 42, a drive device 43, an auxiliary storage device 44, a memory device 45, a Central Professing Unit (CPU) 46, and a network connection device 47. These are mutually connected via a system bus B.

The input device 41 is provided for a user or the like to run programs and input various operation signals, and includes a keyboard, a pointing device such as a mouse or the like. The output device 42 includes a display for displaying various windows, data or the like necessary for operating the computer which carries out processes of the embodiment of the present invention. The output device 42 can display execution transit, results, or the like of the program with a control program installed in the CPU 46. The input device 41 and the output device 42 may be an integrated input and output unit such as a touch panel. In this case, a user's finger, a pen-type input device, and so on may be used to touch the touch panel.

In the embodiment of the present invention, the execution program installed in the computer may be provided by a USB memory, a portable recording medium 48 such as a CD-ROM, and so on. The recording medium 48 having the execution program recorded on it may be mounted on the drive device 43. The execution program recorded in the recording medium 48 may be installed in the auxiliary storage device 44 via the drive device 43.

The auxiliary storage device 44 is a storage means such as a hard disk. The auxiliary storage device 44 can store the execution program of the embodiment of the present invention, and the control program installed on the computer, and so on, thereby enabling to input or output these when necessary.

The memory device 45 stores the execution program which is read out of the auxiliary storage device 44 by the CPU 46, and so on with the CPU 46. The memory device 45 may be a Read Only Memory (ROM), a Random Access Memory (RAM), and so on.

The CPU 46 controls entire processes of the computer such as various calculations and inputs and outputs of data to and from various portions in the hardware configuration in order to realize various processes of the application evaluation with the control program such as an operating system (OS) and the execution program stored in the memory device 45. The various information or the like necessary for running the program may be obtained from the auxiliary storage device 44. The results of the execution may be stored in the auxiliary storage device 44.

When the network connecting device 47 is connected to a communication network or the like, the network connecting device 47 may obtain the execution program from another terminal connected to the communication network, or provide execution results obtained by carrying out the execution program or the execution program itself of the embodiment of the present invention to the other terminal and so on. Further, the network connection device 47 may acquire SPF evaluation values of external dermatological medications such as cosmetic products applied to skin substitute substrates from outer terminals connected to the network.

With the above-mentioned hardware configuration, the evaluation processes of the embodiments of the present invention can be carried out. Further, by installing the program in the general purpose computer and so on, the application evaluating process of the embodiments of the present invention can be easily realized.

(Application evaluating procedure)

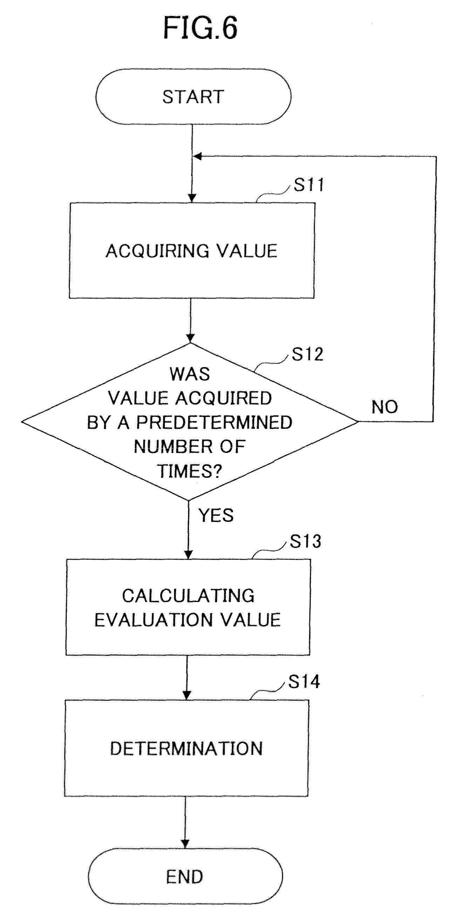

Next, a application evaluating procedure of the embodiments of the present invention is described. FIG. 6 illustrates an example of the application evaluating procedure of the embodiments.

Referring to FIG. 6, values for evaluating permeation characteristics and/or reflection characteristics of ultraviolet radiation are obtained in step S11, and it is determined that a predetermined number of the values is acquired in step S12. If the predetermined number of the values is not acquired in NO of step S12, a value acquiring process of step S11 is carried out. In step S12, if the predetermined number of the values is acquired in YES of step S12, the evaluation value is calculated in step S13 and the determination is done based on the calculated evaluation value in step S14.

(Examples of evaluations with embodiments)

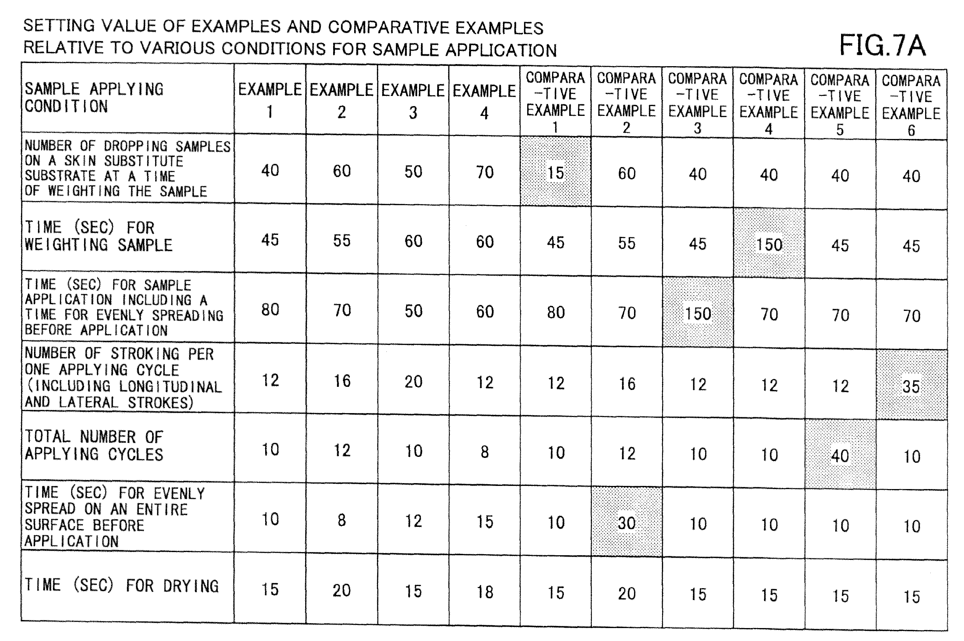

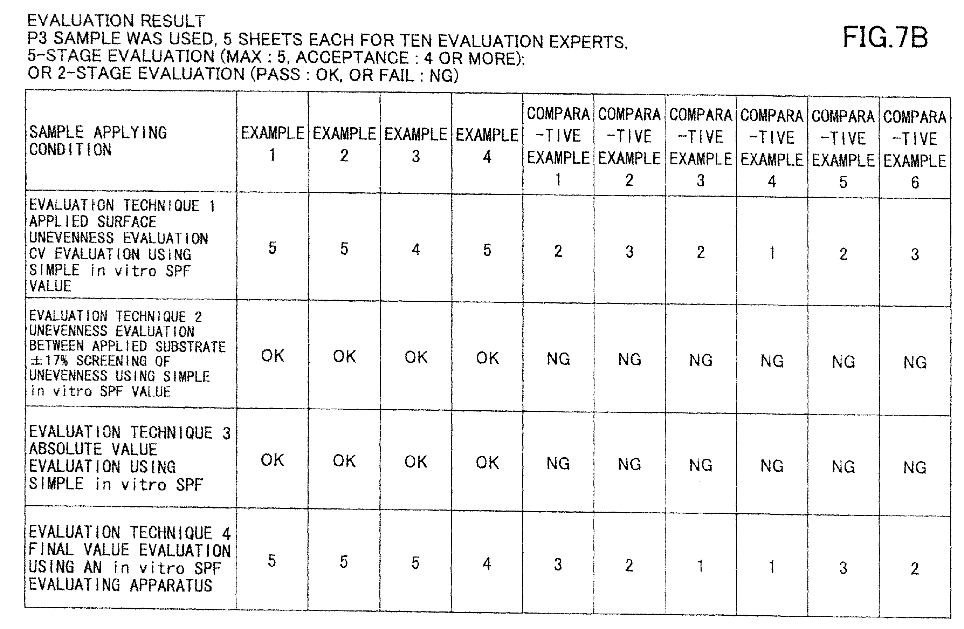

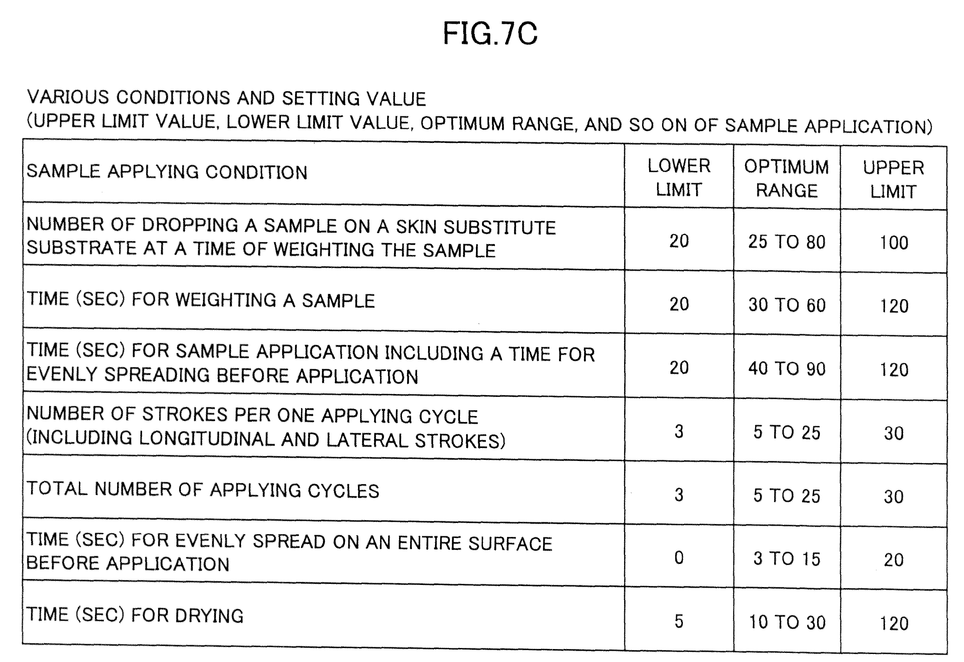

Next, examples of evaluations in the embodiments are described. FIG. 7A, FIG. 7B, and FIG. 7C illustrate evaluation contents of the embodiments.

[Evaluation Method 1: Applied surface unevenness evaluation]

Evaluation technique 1 of applied surface unevenness evaluation is described. The applied surface unevenness evaluation is provided to carry out CV evaluation of evenness in a surface using simple in vitro SPF values.

The CV value can be obtained by dividing a standard deviation by a mean value. The simple in vitro SPF value is acquired by simply calculating ultraviolet radiation protection ability in consideration of an erythema action spectrum based on a ratio between relative erythema effectiveness of a skin or the like not applied with a sample and relative erythema effectiveness of a skin or the like applied with a sample acquired from a measurement for spectral transmission factors. The difference of the in vivo SPF value from the simple in vitro SPF value is that a photodegradation phenomenon is considered in the in vivo SPF value.

In Evaluation technique 1, a predetermined sample is used, the number of evaluation experts is 10, the number of skin substitute substrates to be evaluated is 5 per each evaluation expert, and a 5-stage evaluation (5 is the best point) is used. The sample is, for example, P3 which is a standard sample described in International SPF Test Method 2006. The skin substitute substrate is, for example, an acrylic material having an arithmetic average surface roughness of about 15 to 25 micron, a thickness of 0.2 to 5 mm, a length of 50 mm, and a width of 50 mm.

Sample applying conditions are, for example, at least one of "the number of dropping a sample on the skin substitute substrate at a time of weighing the sample", "the time (sec) for weighing a sample", "time (sec) for sample application including a time for evenly spreading before application", "the number of strokes per one applying cycle (including longitudinal and lateral strokes)", "total number of applying cycles", "time (sec) for evenly spread on an entire surface before application", and "time (sec) for drying after application". The values for the above conditions are as described in Examples 1 to 4 Comparative Examples 1 to 6 illustrated in FIG. 7A, FIG. 7B, and FIG. 7C. Specifically, setup values for the various sample applying conditions in Examples 1 to 4 and Comparative Examples 1 to 6 are illustrated in FIG. 7A. Referring to FIG. 7B, the above P3 sample is used. According to the setup values for the various sample applying conditions in Examples 1 to 4 and Comparative Examples 1 to 6, the number of evaluation experts is 10, the number of skin substitute substrates to be evaluated is 5 per each evaluation expert, and a 5-stage evaluation (5 is the best point; and 4 or more means pass) or a 2-stage evaluation of OK (pass) and NG (fail) is used. In the above applying conditions, setup values such as an upper limit, a lower limit, and an optimum range may be set as illustrated in FIG. 7C. Then, it is possible to evaluate by comparing the setup values with the values in Examples 1 to 4 and Comparative Examples 1 to 6.

A application evaluating apparatus used in Evaluation technique 1 is, for example, spectrophotometer U-4100 manufactured by Hitachi, Ltd.). An evaluating spectrum to be used may be a spectral transmission spectrum. An evaluating wavelength is between 290 to 400 nm with a wavelength step (interval) of 1 to 5 nm, preferable 1 nm.

Measuring positions on the surface of a skin substitute substrate may be dispersed. For example, predetermined five positions like the dot arrangement of No. 5 of a die (four corner positions and one center position at around a cross-point of lines connecting the four corners). The number of the measurement is once for each one of the positions.

Further, data may be analyzed from the evaluating spectrum based on a xenon arc ultraviolet radiation spectrum disclosed in Non-Patent Document 3 (International Sun Protection Factor Test Method, (C.O.L.I.P.A.-J.C.I.A.-C.T.F.A.S.A.-C.T.F.A. ),May 2006, International SPF Test Method 2006). The xenon arc ultraviolet radiation spectrum disclosed in Non-Patent Document 3 is multiplied with spectral transmission factors of the evaluating spectrum and further weighed by erythema factors disclosed in CIE-1987. Then, the above results are integrated through the wavelengths of 290 to 400 nm to acquire a relative erythema effectiveness.

For example, the simple in vitro SPF is calculated by dividing a relative erythema effectiveness for the skin or the like not applied with the sample by relative erythema effectiveness for the skin or the like applied with the sample.

For example, the data may be processed based on the simple in vitro SPF by calculating a mean value of values of the five points per a skin substitute substrate applied with a sample and obtain a CV value (i.e., a value obtained by dividing a standard deviation with a mean value) based on the calculated mean value and the standard deviation of the values of five points.

Determination criteria of the CV value of 10 persons may be a 5-stage evaluation, in which 5 is the best. When the CV value is 30% or more, the evaluation value is 1. When the CV value is 25% or more and less than 30%, the evaluation value is 2. When the CV value is 20% or more and less than 25%, the evaluation value is 3. When the CV value is 15% or more and less than 20%, the evaluation value is 4. When the CV value is less than 15%, the evaluation value is 5.

(Evaluation technique 2: unevenness evaluation between applied substrates)

Next, Evaluation technique 2 which is an unevenness evaluation between applied substrates is described. This evaluating method carries out a 17% screening for the simple in vitro SPF values.

In Evaluation technique 2, the above mentioned P3 sample is used. The number of evaluation experts is 10, the number of skin substitute substrates to be evaluated is 5 per each evaluation expert, and a 2-stage evaluation of OK (pass) and NG (fail) is used.

Conditions for the sample, skin substitute substrates, the number of the skin substitute substrates, and sample applying conditions may be similar to Evaluation technique 1. Further, an evaluating apparatus, an evaluating spectrum, evaluating wavelengths, a wavelength step (interval), measuring position on the skin substitute substrate, the number of measurements, data analysis from the evaluating spectrum, a calculation method of the simple in vitro SPF may be similar to the conditions in Evaluation technique 1.

For example, the data may be processed based on the simple in vitro SPF by calculating a mean value of values of the five points per the one skin substitute substrate applied with the sample and obtain a CV value (i.e., a value obtained by dividing a standard deviation with a mean value) based on the calculated mean value and the standard deviation of the values of five points.

Next, data of which CV value is 20% or less are determined to be effective data. The mean values of the effective data are obtained for each plate (e.g. a skin substitute substrate). Whereafter, a mean value among the plates is obtained. Further, based on the mean value, a standard deviation obtained therefrom, and the number of the effective data, a 95% confidence interval (95% CI) is obtained. Eventually, when the 95% confidence interval (95% CI) indicates a CIn' [%] representing less than 17% of a mean simple in vitro SPF, pass (OK) is determined. When the 95% confidence interval (95% CI) indicates a Cin' [%] representing 17% or more of the mean simple in vitro SPF, fail (NG) is determined.

(Calculation technique used in Evaluation technique 2)

The calculation technique used in Evaluation technique 2 is described next. An SPF test is started by testing a sample under test using skin substitute substrates as many as n' where n' is at least 10. At the initial stage, a provisional mean value of in vitro SPF (i.e., in vitro SPFn') of the skin substitute substrates as many as n' becomes SPFn' = (ΣSPFi)/n'. The standard deviation (s) is as follows:

$$s=\sqrt{[(\Sigma(SPFi^2)-((\Sigma SPFi)^2/n))/(n-1)]}=[(\Sigma(SPFi^2)-((\Sigma SPFi)^2/n))/(n-1)]^{1/2}$$

The 95% confidence interval (95% CI) of the mean SPF is represented by the following formula.

$$95\%CIn'=SPFn'-cn' \sim SPFn'+cn'$$

$$(SPFn'-cn'\leq95\%CIn'\leq SPFn'+cn')$$

The above cn' is calculated as follows.

$$cn'=tn'\times sn'/\sqrt{n'}=tn'\times sn'/(n')^{1/2}$$

The sn' is the standard deviation of the skin substitute substrate as many as n' at the initial stage.

$$sn'=\sqrt{[(\Sigma(SPFi^2)-((\Sigma SPFi)^2/n'))/(n'-1)]}=[(\Sigma(SPFi^2)-((\Sigma SPFi)^2/n'))/(n'-1)]^{1/2}$$

$$CIn'[\%]=100\times cn'/SPFn'$$

where n' is the total amount of the effective data, and tn' is obtained from 'two-sided' Student-t distribution table at p=0.05 in the degree of freedom ν=(n-1). For a table calculation, a value t in the t distribution can be modeled by the following formula.

$$t=2.03+12.7/n^{1.75} \quad (n\geq4)$$

If the calculated provisional CIn' [%] is less than 17% of the provisional in vitro SPFn', pass (OK) is determined. If the calculated provisional CTn' [%] is 17% or more, the skin substitute substrates are added to continue the test until the calculated provisional CIn' [%] becomes 17% or less. Eventually, if the calculated provisional CIn' [%] does not become 17% or less after the test using skin substitute substrates as many as 30 (thirsty), the test is stopped and redone.

(Evaluation technique 3: absolute value evaluation using simple in vitro SPF)

Next, Evaluation technique 3 being an absolute value evaluation using the simple in vitro SPF, is described. In Evaluation technique 3, the above mentioned P3 sample is used. The number of evaluation experts is 10, the number of skin substitute substrates to be evaluated is 5 per each evaluation expert, and the 5-stage evaluation of OK (pass) and NG (fail) is used.

Conditions for the sample, skin substitute substrates, the number of the skin substitute substrates and sample applying conditions may be similar to Evaluation technique 1. Further, an evaluating apparatus, an evaluating spectrum, evaluating wavelengths, a wavelength step (interval), measuring position on the skin substitute substrate, the number of measurements, data analysis from the evaluating spectrum, a calculation method of the simple in vitro SPF may be similar to the conditions in Evaluation technique 1.

In processing data based on the simple in vitro SPF, a mean value of values at the five points per the one skin substitute substrate applied with the sample is calculated. When the mean values in the skin substitute substrates as many as 10 or more are obtained, it is determined whether pass is given relative to criterion value range for the mean values. Specifically, pass of the mean values in the skin substitute substrates as many as 10 or more is checked relative to the criterion value range for the mean values.

(Evaluation technique 4: final evaluation method)

Next, Evaluation technique 4 being a final evaluation method is described. In Evaluation technique 4, a final value evaluation using an in vitro SPF evaluating apparatus is carried out.

In Evaluation technique 4, the above mentioned P3 sample is used. The number of evaluation experts is 10, the number of skin substitute substrates to be evaluated is 5 per each evaluation expert, and the 5-stage evaluation (5 is the best point) is used.

Conditions for the sample, skin substitute substrates, the number of the skin substitute substrates, and sample applying conditions may be similar to Evaluation technique 1. A application evaluating apparatus used in Evaluation technique, 4 is, for example, spectrophotometer U-4100 manufactured by Hitachi, Ltd.). An evaluating spectrum to be used may be a spectral transmission spectrum. An evaluating wavelength is between 290 to 400 nm with a wavelength step (interval) of 1 to 5 nm, preferably 1 nm.

For example, one measuring position on the skin substitute substrate may be at a predetermined position such as a center portion. The number of the measurement is once for each one of the positions. In the application evaluating apparatus, a long measurement time is required, and a drying time after application is preferably constant. Therefore, it is measured once.

The calculation method of an in vitro SPF in Evaluation technique 4 is based on an evaluation algorithm disclosed in the document of "Y.Miura et.al.,Photochemistry and Photobiology, 2008, 84:1569-1575". With this calculation method, a light is continuously emitted to the sample, and the emitted light is time-integrated until an endpoint where it becomes 1 minimal erythema dose (MED).

A mean value of in vitro SPF values in five skin substitute substrates applied with a sample is calculated as a data processing based on the in vitro SPF.

When a mean value in 10 persons is used, exemplary determination criteria of the in vitro SPF values may be determined as follows. For example, when the mean value is 11.7 or less or 20.7 or more, the evaluation value thereof may be determined as "1". When the mean value is 11.7 or more and less than 12.7 or 19.7 or more and less than 20.7, the evaluation value thereof may be determined as "2". When the mean value is 12.7 or more and less than 13.7 or 18.7 or more and less than 19.7, the evaluation value thereof may be determined as "3". When the mean value is 13.7 or more and less than 15.2 or 17.2 or more and less than 18.7, the evaluation value thereof may be determined as "4". When the mean value is 15.2 or more and less than 17.2, the evaluation value thereof may be determined as "5".

The above calculation methods are not specifically limited. Based on the sample applying conditions of Examples 1 to 4 and Comparative Examples 1 to 6, the evaluation results by the evaluation techniques 1 to 4 illustrated in FIG. 7B are obtainable. For example, in a case of a 5-stage evaluation, 4 or more means pass. In a case of a 2-stage evaluation, OK (pass) or NG (fail) is obtainable.

In the various conditions of the various sample applying conditions, an upper limit, a lower limit, and an optimum range may be set as illustrated in FIG. 7C, and the evaluation may be carried out by comparing the actually measured values with the upper limit, the lower limit, and the optimum range. With this, in Examples 1 to 4 and Comparative Examples 1 to 6 illustrated in FIG. 7A, when the value exceeds the upper limit or becomes lower than the lower limit, the value may be highlighted to easily report fail (NG).

By using the above evaluating method, it is possible to evenly evaluate the application with high accuracy. As described, by providing the training method, it is possible to improve application repeatability in applying the external dermatological medications.

With the embodiments, it is possible to provide applicators with clear training procedures for improving application repeatability. Further, in the embodiments by providing the following tools for the training or the like, the applying trainings are formalized as knowledge for the applicators and the skills of the applicators can be easily and accurately determined.

(Training procedures for improving application repeatability of the external dermatological medications)

Hereinafter, training procedures for improving application repeatability of the external dermatological medications are described. An applicator first reads a manual of a standardized application method. Next, the applicator watches a motion picture or a demonstration demonstrating an equalized application method. Next, the applicator attends a demonstration lecture by an instructor of the application method and learns the application method to improve the applicator's skill. Meanwhile, the applicator experiences n-service training of the application method.

<Determining technique>

Next, a determining technique of the embodiments is described. In the determining technique, values corresponding to a sample in vitro SPF without considering the photodegradation acquired by using a spectrophotometer and so on or in vitro SPF values acquired by an in vitro SPF measuring apparatus and so on are compared with criterion value ranges. For example, if the unevenness of the data is in the criterion value range, the application repeatability is determined to be sufficient. If the acquired spectral SPF values are within the criterion value range, the predictions accuracy is determined to be sufficient. When both of the application repeatability and the predictions accuracy are determined to be sufficient, pass is given to the applicator to end the applicator is training.

Next, based on the predetermined training sheet, the actual in vitro SPF test is started after the pass of the above contents. By passing the test, data having high application repeatability is obtainable.

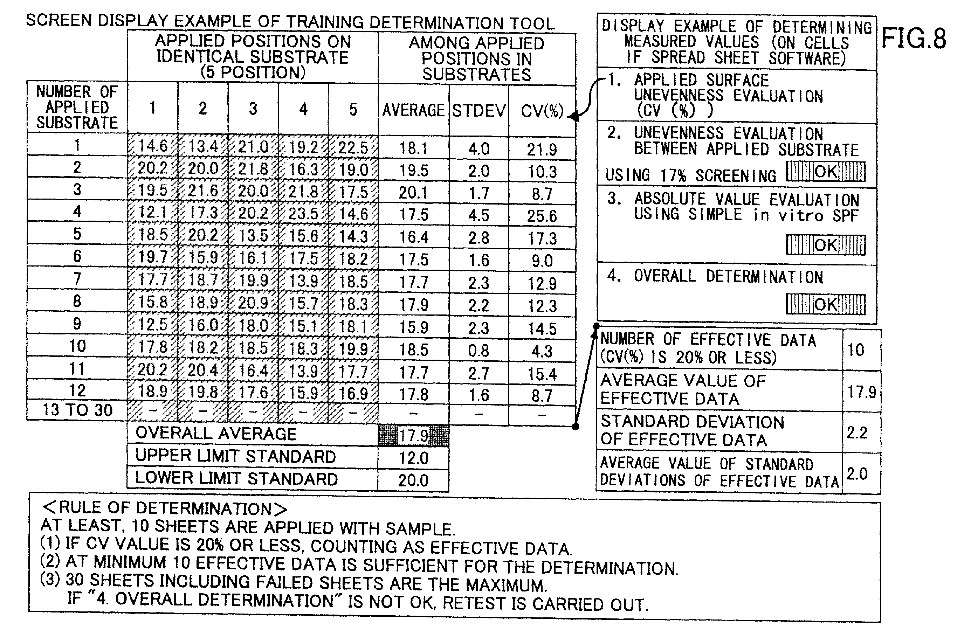

FIG. 8 illustrates measurement results of the in vitro SPF values at 5 applied positions on each applied substrate, and an exemplary display of measured values with the training determination tool of the embodiments. The measurement results of the in vitro SPF values at 5 applied positions on each applied substrate, and the display of measured values with the training determination tool of the embodiments may be realized by commercially available spreadsheet software or a uniquely developed application. Referring to FIG. 8, the above evaluation technique 1 is used in skin substitute substrates (plates) as many as 30 sheets to acquire measurement results of the simple in vitro SPF values at 5 positions of each applied substrate. Applied surface unevenness evaluation values for evaluating evenness within substrate at five positions are acquired. The average value (Average), the sample standard deviation (STDEV), the unevenness evaluation value (CV value [%]) on the applied surface calculated by the above evaluation technique 1 are calculated. For example, a part of the applied surface unevenness evaluation values having the CV values of 20% or less is used for the determination between "OK" and "NG", and so on.

Referring to the table in FIG. 8, the numbers of applied substrate 13 to 30 are collectively integrated in one line for simplicity. However, it is intended to deploy the line of the numbers 13 to 30 into other lines of the numbers 13 to 30. The measured values of the applied substrates of the numbers 13 to 30 are omitted and instead indicated by "-".

Referring to FIG. 8, the samples are evaluated by comparing the values acquired by the above value acquiring unit 34 with an overall average, an upper limit standard, and a lower limit standard set up in the table of FIG. 8. The overall average is not limited to consider the effective data, and may be obtained by adding the averages of the values at the 5 applied position for all the applied substrates of the numbers 1 to 30, and dividing the added averages by 30. The upper limit standard and the lower limit standard are previously set as allowable simple in vitro SPF values.

Referring to FIG. 8, it is possible to display the number of effective data, an average value of the effective data, a standard deviation of the effective data, an average value of the standard deviations of the effective data, and so on. Assuming that the number of the effective data is 10 corresponding to the numbers 2, 3, and 5 to 12 of the applied substrates, the corresponding average value of the effective data, standard deviation of the effective data, and average value of the standard deviations of the effective data are illustrated in FIG. 8.

Further, as a measured value determination, "1. APPLIED SURFACE UNEVENNESS EVALUATION", "2. UNEVENNESS EVALUATION BETWEEN APPLIED SUBSTRATES", "3. ABSOLUTE VALUE EVALUATION USING SIMPLE IN VITRO SPF", "4. OVERALL DETERMINATION" and so on may be set and used for the determination.

For example, the determination result acquired by determining "OK" or "NG" using the effective data having the CV values of 20% or less is displayed "OK" or "NG" on cells of the spreadsheet software.

Further, the overall "meanvalue", "standard deviation", "number of trainees (applicators) "and so on may be displayed. Further, byhighlightingimportant portions as the calculation results, it is possible to easily understand the determination results.

An exemplary determination tool of training results is also illustrated in FIG. 8. With the determination tool, results of the training can be evaluated by determining the training as follows. At least 10 sheets are obtained by the application. (1) If the CV value is 20% or less, the corresponding data are counted as the effective data. (2) At minimum, 10 effective data are used for the determination. (3) The maximum number of the sheets is 30 including the rejected sheets. At this time, if "4. OVERALL DETERMINATION" is not OK, a retest is carried out.

According to the embodiments of the present invention, when predetermined portions of a skin, a skin substitute substrate and so on are applied with an external dermatological medication such as a cosmetic product, knowhow and experience of skilled personnel are formalized as knowledge. Then, the embodiments systematically provide a clear method and a training procedure so that a person having no experience of applying an external dermatological medication to a skin substitute substrate and so on can attain high application repeatability within a very short time. With this, application repeatability is enhanced to thereby resultantly enhance application repeatability of data of in vitro SPF measurements and so on.

Further, the content of the training determination tool is not limited to the above. For example, a determination tool corresponding to the above evaluation technique 2 may be provided. Further, the evaluation sheet having a macro for executing the above procedure in the training determination tool may be made of spreadsheet software. By inputting data into appropriate area (cell) of the spreadsheet software, effective and easy calculations may be carried out. Thereafter, an accurate evaluation may be carried out based on the calculation results.

With the embodiments of the present invention, it is possible to provide a application method of external dermatological medications in which repeatability of applying the external dermatological medications is improved, an evaluating method of the application method, a application evaluating apparatus, and a application evaluating program. Although, in the embodiments, the evaluating apparatus for evaluating permeation characteristics and/or reflection characteristics four the ultraviolet radiation in the external dermatological medications is used for the application evaluations, the embodiments of the present invention are not limited thereto and other characteristics may be used for determining the application evaluation.

Further, by applying the embodiments of the present invention, the sample can be evenly applied. Therefor, when an identical person carries out an identical application method, by determining unevenness of the sample applied to various skin substitute substrates, it is possible to evaluate easiness of the application of the various skin substitute substrates. Further, by applying the embodiments of the present invention, technical levels of an applicators may also be evaluated based on predetermined criteria.

Although there has been described the embodiments of the present invention, the present invention is not limited to the above embodiment, and various modifications and changes are possible in the scope of the present invention described in the claims.

The international application is based on Japanese Priority Patent Application No. 2009-081400 filed on March 30, 2009, the entire contents of which are hereby incorporated herein by reference.

EXPLANATION OF REFERENCE SYMBOLS

| | |
|---|---|
| 1,16: | a skin substitute substrate |
| 10: | application evaluating apparatus |
| 11: | light source |
| 12: | filter |
| 13: | optical fiber |
| 14: | irradiation port |
| 16: | external dermatological medications |
| 17: | detection port |
| 18: | optical fiber |
| 19: | spectrometer |
| 20: | photodetector |
| 21: | electric signal and application evaluating processing unit (computer) |
| 22: | lock-in amplifier |
| 23: | optical chopper |
| 24: | integrating sphere |
| 31: | input unit |
| 32: | output unit |
| 33: | storage unit |
| 34: | value acquiring unit |
| 35: | evaluation value calculating unit |
| 36: | determining unit |
| 37: | sending and receiving unit |
| 38: | control unit |
| 41: | input device |
| 42: | output device |
| 43: | drive device |

44: auxiliary storage device
45: memory device
46: CPU
47: network connection device
48: recording medium

**Claims**

1. A method of applying an external dermatological medication to a skin or a skin substitute substrate for evaluating characteristics of the external dermatological medication acquired by irradiating the skin or the skin substitute substrate with light, the method comprising:

   a first applying step of spreading the external dermatological medication in one direction on the skin or the skin substitute substrate from a side surface of the skin or the skin substitute substrate; and
   a second applying step of spreading the external dermatological medication in a direction perpendicular to the one direction on the skin or the skin substitute substrate,
   wherein the first applying step and the second applying step are repeated by a predetermined number of times.

2. The method of applying the external dermatological medication to the skin or the skin substitute substrate according to claim 1,
   wherein a time for the first applying step and the second applying step multiplied by the predetermined number of times results in about 30 seconds to about 90 seconds after starting the first applying step or the second applying step, and a number of strokes for applying to the skin or the skin substitute substrate per about one second is 0.5 to 5 and one cycle including the first applying step and the second applying step is about 3 seconds to about 20 seconds.

3. The evaluating method of the skin or the skin substitute substrate obtained by the method of applying the external dermatological medication to the skin or the skin substitute substrate according to claim 1, the evaluating method comprising:

   acquiring a value by changing a transmitted light intensity or a transmittance, or a reflected light intensity or a reflectance, which are acquired by irradiating the skin or the skin substitute substrate with the light;
   calculating an evaluation value for carrying out at least one of a applied surface unevenness evaluation, an unevenness evaluation between applied substrates, an absolute value evaluation using a simple in vitro SPF, and a final value evaluation using an in vitro SPF evaluating apparatus by acquiring the values acquired by the acquiring the value for plural times; and
   determining whether applying the external dermatological medication to the skin or the skin substitute substrate is accurately carried out based on the evaluation value calculated by the calculating the evaluation value.

4. The evaluating method according to claim 3,
   wherein the calculating the evaluation value calculates a CV evaluation value of distribution of the simple in vitro SPFs as the unevenness evaluation between applied substrates.

5. The evaluating method according to claim 3,
   wherein a 17% screening of distribution of the simple in vitro SPF values is carried out as the unevenness evaluation between applied substrates.

6. An application evaluating apparatus for evaluating characteristics of an external dermatological medication acquired by irradiating the external dermatological medication with light after applying the external dermatological medication to the skin or a skin substitute substrate, the application evaluating apparatus comprising:

   a value acquiring unit configured to acquire a value by changing a transmitted light intensity or a transmittance, or a reflected light intensity or a reflectance, which are acquired by irradiating the skin or the skin substitute substrate with the light;
   an evaluation value calculating unit configured to calculate an evaluation value for carrying out at least one of a applied surface unevenness evaluation, an unevenness evaluation between applied substrates, an absolute value evaluation using a simple in vitro SPF, and a final value evaluation using an in vitro SPF evaluating apparatus by acquiring the values acquired by the value acquiring unit for plural times; and
a determining unit configured to determine whether applying the external dermatological medication to the skin or the skin substitute substrate is accurately carried out based on the evaluation value calculated by the calculating the evaluation value.

7. The application evaluating apparatus according to claim 6,
wherein the evaluation value calculating unit calculates a CV evaluation value of distribution of the simple invitro SPFs as the unevenness evaluationbetween applied substrates.

8. The application evaluating apparatus according to claim 6,
wherein the evaluation value calculating unit carries out a 17% screening of distribution of the simple in vitro SPF values is carried out as the unevenness evaluation between applied substrates.

9. An application evaluating program for evaluating characteristics of an external dermatological medication acquired by irradiating the external dermatological medication with light after applying the external dermatological medication to the skin or a skin substitute substrate, the evaluating program causing a computer to function as:

   a value acquiring unit configured to acquire a value by changing a transmitted light intensity or a transmittance, or a reflected light intensity or a reflectance, which are acquired by irradiating the skin or the skin substitute substrate with the light;
   an evaluation value calculating unit configured to calculate an evaluation value for carrying out at least one of a applied surface unevenness evaluation, an unevenness evaluation between applied substrates, an absolute value evaluation using a simple in vitro SPF, and a final value evaluation using an in vitro SPF evaluating apparatus by acquiring the values acquired by the value acquiring unit for plural times; and
   a determining unit configured to determine whether applying the external dermatological medication to the skin or the skin substitute substrate is accurately carried out based on the evaluation value calculated by the calculating the evaluation value.

10. The application evaluating program according to claim 9,
wherein the evaluation value calculating unit calculates a CV evaluation value of distribution of the simple in vitro SPFs as the unevenness evaluation between applied substrates.

11. The application evaluating program according to claim 9,
wherein the evaluation value calculating unit carries out a 17% screening of distribution of the simple in vitro SPF values is carried out as the unevenness evaluation between applied substrates.

# FIG.1

```
        ┌─────────────┐
        │    START    │
        └─────────────┘
               │
               ▼
```

S01

```
┌───────────────────────────────┐
│        PREPROCESSING          │
└───────────────────────────────┘
               │
               ▼
```

S02

```
┌───────────────────────────────┐
│       WEIGHTING SAMPLE        │
└───────────────────────────────┘
               │
               ▼
```

S03

```
┌───────────────────────────────┐
│    FIXING SKIN SUBSTITUTE     │
│          SUBSTRATE            │
└───────────────────────────────┘
               │
               ▼
```

S04

```
┌───────────────────────────────┐
│  SAMPLE SPOTS ARE SPREAD      │
│  OUT BY DAUBING ALONG SMALL   │
│  CIRCLES MANY TIMES TO EVENLY │
│    APPLY TO ENTIRE SURFACE    │
└───────────────────────────────┘
               │
               ▼
```

S05

```
┌───────────────────────────────┐
│   SAMPLE SPOTS ARE SPREAD     │
│      OUT IN ONE DIRECTION     │
│      FROM A SIDE SURFACE      │
└───────────────────────────────┘
               │
               ▼
```

S06

```
┌───────────────────────────────┐
│ SAMPLE SPOTS ARE SPREAD OUT IN│
│  A DIRECTION PERPENDICULAR TO │
│  THE ONE DIRECTION OF STEP S05│
└───────────────────────────────┘
               │
               ▼
```

S07

```
            ╱─────────────╲
           ╱      WAS       ╲
          ╱ SPREADING CARRIED ╲    NO
          ╲ OUT BY A PREDETERMINED ╱───┐
           ╲   NUMBER OF TIMES    ╱    │
            ╲        ?          ╱     │
              ╲──────────────╱        │
               │ YES                  │
               ▼                      │
```

S08

```
┌───────────────────────────────┐
│           DRYING              │
└───────────────────────────────┘
               │
               ▼
        ┌─────────────┐
        │     END     │
        └─────────────┘
```

# FIG.2

[I]

[II]

REPEATING [I] TO [II] BY A PREDETERMINED NUMBER OF TIMES

EP 2 416 144 A1

# FIG.3

# FIG.4

EP 2 416 144 A1

# FIG.5

```
     45              46                        47
MEMORY UNIT       CPU              NETWORK
                                CONNECTION DEVICE
     ↕             ↕                        ↕
─────────────────────────────────────────────────
B          ↕             ↕            ↕            ↕
        41            42           43           44
   INPUT DEVICE    OUTPUT      DRIVE DEVICE   AUXILIARY
                   DEVICE                     STORAGE
                                              DEVICE
                                  ↕
                                  48
                              RECORDING
                               MEDIUM
```

# FIG.6

START

S11

ACQUIRING VALUE

S12

WAS
VALUE ACQUIRED
BY A PREDETERMINED
NUMBER OF
TIMES?

NO

YES

S13

CALCULATING
EVALUATION VALUE

S14

DETERMINATION

END

SETTING VALUE OF EXAMPLES AND COMPARATIVE EXAMPLES
RELATIVE TO VARIOUS CONDITIONS FOR SAMPLE APPLICATION

FIG.7A

| SAMPLE APPLYING CONDITION | EXAMPLE 1 | EXAMPLE 2 | EXAMPLE 3 | EXAMPLE 4 | COMPARA-TIVE EXAMPLE 1 | COMPARA-TIVE EXAMPLE 2 | COMPARA-TIVE EXAMPLE 3 | COMPARA-TIVE EXAMPLE 4 | COMPARA-TIVE EXAMPLE 5 | COMPARA-TIVE EXAMPLE 6 |
|---|---|---|---|---|---|---|---|---|---|---|
| NUMBER OF DROPPING SAMPLES ON A SKIN SUBSTITUTE SUBSTRATE AT A TIME OF WEIGHTING THE SAMPLE | 40 | 60 | 50 | 70 | 15 | 60 | 40 | 40 | 40 | 40 |
| TIME (SEC) FOR WEIGHTING SAMPLE | 45 | 55 | 60 | 60 | 45 | 55 | 45 | 150 | 45 | 45 |
| TIME (SEC) FOR SAMPLE APPLICATION INCLUDING A TIME FOR EVENLY SPREADING BEFORE APPLICATION | 80 | 70 | 50 | 60 | 80 | 70 | 150 | 70 | 70 | 70 |
| NUMBER OF STROKING PER ONE APPLYING CYCLE (INCLUDING LONGITUDINAL AND LATERAL STROKES) | 12 | 16 | 20 | 12 | 12 | 16 | 12 | 12 | 12 | 35 |
| TOTAL NUMBER OF APPLYING CYCLES | 10 | 12 | 10 | 8 | 10 | 12 | 10 | 10 | 40 | 10 |
| TIME (SEC) FOR EVENLY SPREAD ON AN ENTIRE SURFACE BEFORE APPLICATION | 10 | 8 | 12 | 15 | 10 | 30 | 10 | 10 | 10 | 10 |
| TIME (SEC) FOR DRYING | 15 | 20 | 15 | 18 | 15 | 20 | 15 | 15 | 15 | 15 |

EP 2 416 144 A1

EVALUATION RESULT
P3 SAMPLE WAS USED, 5 SHEETS EACH FOR TEN EVALUATION EXPERTS,
5-STAGE EVALUATION (MAX : 5, ACCEPTANCE : 4 OR MORE);
OR 2-STAGE EVALUATION (PASS : OK, OR FAIL : NG)

FIG.7B

| SAMPLE APPLYING CONDITION | EXAMPLE 1 | EXAMPLE 2 | EXAMPLE 3 | EXAMPLE 4 | COMPARA -TIVE EXAMPLE 1 | COMPARA -TIVE EXAMPLE 2 | COMPARA -TIVE EXAMPLE 3 | COMPARA -TIVE EXAMPLE 4 | COMPARA -TIVE EXAMPLE 5 | COMPARA -TIVE EXAMPLE 6 |
|---|---|---|---|---|---|---|---|---|---|---|
| EVALUATION TECHNIQUE 1 APPLIED SURFACE UNEVENNESS EVALUATION CV EVALUATION USING SIMPLE in vitro SPF VALUE | 5 | 5 | 4 | 5 | 2 | 3 | 2 | 1 | 2 | 3 |
| EVALUATION TECHNIQUE 2 UNEVENNESS EVALUATION BETWEEN APPLIED SUBSTRATE ±17% SCREENING OF UNEVENNESS USING SIMPLE in vitro SPF VALUE | OK | OK | OK | OK | NG | NG | NG | NG | NG | NG |
| EVALUATION TECHNIQUE 3 ABSOLUTE VALUE EVALUATION USING SIMPLE in vitro SPF | OK | OK | OK | OK | NG | NG | NG | NG | NG | NG |
| EVALUATION TECHNIQUE 4 FINAL VALUE EVALUATION USING AN in vitro SPF EVALUATING APPARATUS | 5 | 5 | 5 | 4 | 3 | 2 | 1 | 1 | 3 | 2 |

EP 2 416 144 A1

# FIG.7C

VARIOUS CONDITIONS AND SETTING VALUE
(UPPER LIMIT VALUE, LOWER LIMIT VALUE, OPTIMUM RANGE, AND SO ON OF SAMPLE APPLICATION)

| SAMPLE APPLYING CONDITION | LOWER LIMIT | OPTIMUM RANGE | UPPER LIMIT |
|---|---|---|---|
| NUMBER OF DROPPING A SAMPLE ON A SKIN SUBSTITUTE SUBSTRATE AT A TIME OF WEIGHTING THE SAMPLE | 20 | 25 TO 80 | 100 |
| TIME (SEC) FOR WEIGHTING A SAMPLE | 20 | 30 TO 60 | 120 |
| TIME (SEC) FOR SAMPLE APPLICATION INCLUDING A TIME FOR EVENLY SPREADING BEFORE APPLICATION | 20 | 40 TO 90 | 120 |
| NUMBER OF STROKES PER ONE APPLYING CYCLE (INCLUDING LONGITUDINAL AND LATERAL STROKES) | 3 | 5 TO 25 | 30 |
| TOTAL NUMBER OF APPLYING CYCLES | 3 | 5 TO 25 | 30 |
| TIME (SEC) FOR EVENLY SPREAD ON AN ENTIRE SURFACE BEFORE APPLICATION | 0 | 3 TO 15 | 20 |
| TIME (SEC) FOR DRYING | 5 | 10 TO 30 | 120 |

EP 2 416 144 A1

SCREEN DISPLAY EXAMPLE OF TRAINING DETERMINATION TOOL

| NUMBER OF APPLIED SUBSTRATE | APPLIED POSITIONS ON IDENTICAL SUBSTRATE (5 POSITION) | | | | | AMONG APPLIED POSITIONS IN SUBSTRATES | | |
|---|---|---|---|---|---|---|---|---|
| | 1 | 2 | 3 | 4 | 5 | AVERAGE | STDEV | CV(%) |
| 1 | 14.6 | 13.4 | 21.0 | 19.2 | 22.5 | 18.1 | 4.0 | 21.9 |
| 2 | 20.2 | 20.0 | 21.8 | 16.3 | 19.0 | 19.5 | 2.0 | 10.3 |
| 3 | 19.5 | 21.6 | 20.0 | 21.8 | 17.5 | 20.1 | 1.7 | 8.7 |
| 4 | 12.1 | 17.3 | 20.2 | 23.5 | 14.6 | 17.5 | 4.5 | 25.6 |
| 5 | 18.5 | 20.2 | 13.5 | 15.6 | 14.3 | 16.4 | 2.8 | 17.3 |
| 6 | 19.7 | 15.9 | 16.1 | 17.5 | 18.2 | 17.5 | 1.6 | 9.0 |
| 7 | 17.7 | 18.7 | 19.9 | 13.9 | 18.5 | 17.7 | 2.3 | 12.9 |
| 8 | 15.8 | 18.9 | 20.9 | 15.7 | 18.3 | 17.9 | 2.2 | 12.3 |
| 9 | 12.5 | 16.0 | 18.0 | 15.1 | 18.1 | 15.9 | 2.3 | 14.5 |
| 10 | 17.8 | 18.2 | 18.5 | 18.3 | 19.9 | 18.5 | 0.8 | 4.3 |
| 11 | 20.2 | 20.4 | 16.4 | 13.9 | 17.7 | 17.7 | 2.7 | 15.4 |
| 12 | 18.9 | 19.8 | 17.6 | 15.9 | 16.9 | 17.8 | 1.6 | 8.7 |
| 13 TO 30 | – | – | – | – | – | – | – | – |
| OVERALL AVERAGE | | | | | | 17.9 | | |
| UPPER LIMIT STANDARD | | | | | | 12.0 | | |
| LOWER LIMIT STANDARD | | | | | | 20.0 | | |

DISPLAY EXAMPLE OF DETERMINING MEASURED VALUES (ON CELLS IF SPREAD SHEET SOFTWARE)

FIG.8

1. APPLIED SURFACE UNEVENNESS EVALUATION (CV (%) )

2. UNEVENNESS EVALUATION BETWEEN APPLIED SUBSTRATE

   USING 17% SCREENING ▓▓OK▓▓

3. ABSOLUTE VALUE EVALUATION USING SIMPLE in vitro SPF

   ▓▓OK▓▓

4. OVERALL DETERMINATION

   ▓▓OK▓▓

| NUMBER OF EFFECTIVE DATA (CV(%) IS 20% OR LESS) | 10 |
|---|---|
| AVERAGE VALUE OF EFFECTIVE DATA | 17.9 |
| STANDARD DEVIATION OF EFFECTIVE DATA | 2.2 |
| AVERAGE VALUE OF STANDARD DEVIATIONS OF EFFECTIVE DATA | 2.0 |

<RULE OF DETERMINATION>
AT LEAST, 10 SHEETS ARE APPLIED WITH SAMPLE.
(1) IF CV VALUE IS 20% OR LESS, COUNTING AS EFFECTIVE DATA.
(2) AT MINIMUM 10 EFFECTIVE DATA IS SUFFICIENT FOR THE DETERMINATION.
(3) 30 SHEETS INCLUDING FAILED SHEETS ARE THE MAXIMUM.
    IF "4. OVERALL DETERMINATION" IS NOT OK, RETEST IS CARRIED OUT.

EP 2 416 144 A1

<div align="center">

**INTERNATIONAL SEARCH REPORT**

</div>

| | International application No. |
|---|---|
| | PCT/JP2010/055729 |

A. CLASSIFICATION OF SUBJECT MATTER
*G01N21/33*(2006.01)i, *A61B5/00*(2006.01)i, *A61K8/00*(2006.01)i

According to International Patent Classification (IPC) or to both national classification and IPC

B. FIELDS SEARCHED

Minimum documentation searched (classification system followed by classification symbols)
G01N21/00-21/61, A61B5/00, A61K8/00

Documentation searched other than minimum documentation to the extent that such documents are included in the fields searched
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | Akira ISHIKUBO et al., "Shinki In Vitro Shigaisen Bogyosei Hyokaho no Kaihatsu", Journal of SCCJ, 20 March 2003 (20.03.2003), vol.37, no.1, pages 10 to 16 | 6-11 |
| A | Ferrero L.et al., Importance of Substrate Roughness for In Vitro Sun Protection Assessment, IFSCC Magazine, 2006.06, Vol.9, No.2, p.97-p.108 | 6-11 |
| A | Weigmann H J, et al., Spectroscopic Characterization of the Sunscreen Efficasy - Basic of a Universal Sunscreen Protection Factor, SOFW Journal, 2006.09.07, Vol.132, No.9, p2, p4-6, p8-10 | 6-11 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 June, 2010 (23.06.10) | 06 July, 2010 (06.07.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/055729

C (Continuation). DOCUMENTS CONSIDERED TO BE RELEVANT

| Category* | Citation of document, with indication, where appropriate, of the relevant passages | Relevant to claim No. |
|---|---|---|
| A | Masako NAGANUMA et al., "Nippon Keshohin Kogyo Rengokai UVA Boshi Koka Sokuteiho Kijun no Kakuritsu", Journal of SCCJ, 20 December 1997 (20.12.1997), vol.31, no.4, pages 420 to 428 | 6-11 |
| A | JP 2008-111834 A (Shiseido Co., Ltd.), 15 May 2008 (15.05.2008), entire text; all drawings & US 2010/0014069 A & EP 2071318 A1 & WO 2008/044596 A1 & KR 10-2009-0060418 A & CN 101523193 A | 6-11 |
| A | JP 05-071927 A (Daicel Chemical Industries, Ltd.), 23 March 1993 (23.03.1993), entire text; all drawings (Family: none) | 6-11 |
| A | JP 08-247746 A (Nireco Corp.), 27 September 1996 (27.09.1996), entire text; all drawings (Family: none) | 6-11 |
| A | JP 2000-002514 A (Nikon Corp.), 07 January 2000 (07.01.2000), entire text; all drawings (Family: none) | 6-11 |
| A | JP 2003-047907 A (Kurabo Industries Ltd.), 18 February 2003 (18.02.2003), entire text; all drawings (Family: none) | 6-11 |
| A | JP 60-068079 A (Nissan Motor Co., Ltd.), 18 April 1985 (18.04.1985), entire text; all drawings (Family: none) | 6-11 |

Form PCT/ISA/210 (continuation of second sheet) (July 2009)

<table>
<tr><td>**INTERNATIONAL SEARCH REPORT**</td><td>International application No.<br>PCT/JP2010/055729</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 1-5
   because they relate to subject matter not required to be searched by this Authority, namely:
   On the basis of the disclosure in paragraph [0003] in the description of the present application "in the case where functions such as UV light-protecting properties are expressed by an application behavior", the disclosure in paragraph [0037] therein (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

| INTERNATIONAL SEARCH REPORT | International application No. |
|---|---|
| | PCT/JP2010/055729 |

Continuation of Box No.II-1 of continuation of first sheet(2)

"a sunscreen (sun-block) cosmetic as an example of the external skin preparation to be applied" and so on, it appears that "an application method" for "applying an external skin preparation to the skin or a skin substitute film" in the inventions of claims 1 to 5 pertains to "methods for treatment of the human body by surgery or therapy, as well as diagnostic methods".

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3337832 B
- JP 2008096151 A
- JP 2008111834 A
- JP 2002048789 A
- JP 2009081400 A

### Non-patent literature cited in the description

- **Ferrero L. et al.** Importance of Substrate Roughness for In vitro Sun Protection Assessment. *IFSCC Magazine,* 2006, vol. 9 (2), 2-13
- COLIPA GUIDELINES, METHOD FOR THE IN VITRO DETERMINATION OF UVA PROTECTION PROVIDED BY SUNSCREEN PRODUCTS. 2007
- **Y.Miura.** *Photochemistry and Photobiology,* 2008, vol. 84, 1569-1575